# EUROPEAN PATENT APPLICATION

(11) **EP 3 835 433 A1**
(43) Date of publication of application: **16.06.2021**
(21) Application number: 19215485.4
(22) Date of filing: 12.12.2019
(51) Int. Cl.: C12Q 1/6886

(54) **NOTCH SIGNALING PATHWAY ACTIVITY AS PROGNOSTIC MARKER IN BLADDER CANCER**

(71) Applicant: Koninklijke Philips N.V., 5656 AG Eindhoven (NL)
(72) Inventor: HOLTZER, Laurentius Henricus Franciscus Maria, 5656 AE Eindhoven (NL); VAN DE STOLPE, Anja, 5656 AE Eindhoven (NL)
(74) Representative: Philips Intellectual Property & Standards

(57) **Abstract**

The invention relates to a method of determining a prognostic score indicating prognosis for a subject diagnosed with bladder cancer. The method comprises receiving an activity of a Notch cellular signaling pathway determined in an urothelial cell sample obtained from the subject. The received activity is compared to one or more reference activities. The prognostic score is determined, wherein the prognostic score is defined such that the prognosis is worse the higher the activity is. The method advantageously allows to draw prognosis with respect to the risk of having or acquiring muscle invasive or metastatic bladder cancer, or with respect to time to metastasis, survival rate or progression of bladder cancer, or risk of relapse of bladder cancer.

## Description

### FIELD OF THE INVENTION

The invention relates to the field of bladder cancer, and more specifically to determining a prognostic score indicating prognosis for a subject diagnosed with bladder cancer and predicting a response score indicating responsiveness of a subject diagnosed with bladder cancer to treatment with an inhibitor of the Notch cellular signaling pathway.

### BACKGROUND OF THE INVENTION

Among men, bladder cancer is the fourth most common cancer and the eighth most common cause of cancer death. Clinical outcome of advanced stage bladder cancer has not significantly improved over the past decades. The general 5-year survival rate for people with bladder cancer is 77%. The 5-year survival rate of people with bladder cancer that has not spread beyond the inner layer of the bladder wall is 95%. If the tumor is invasive but has not yet spread outside the bladder, the 5-year survival rate is 69%. If the cancer extends through the bladder to the surrounding tissue or has spread to nearby lymph nodes or organs, the 5-year survival rate decreases to 35%. If the cancer has spread to distant parts of the body, the 5-year survival rate is as low as 5%. About 4% of people are diagnosed with this stage.

Patient survival can thus be dramatically increased by detecting bladder cancer as early as possible. However, the major clinical problem is that more than 70 % of patients with non-muscle-invasive bladder cancer will have disease recurrence within 2 years of treatment. Extensive long-term surveillance and repeated surgical intervention is needed to prevent progression of early-stage cancers to the more lethal invasive disease. Thus, there is a need for assays that allow early identification of patients that are at risk for metastasis as well as assays that aid treatment choice.

### SUMMARY OF THE INVENTION

In accordance with a first aspect of the present invention, the above problem is solved by a method of determining a prognostic score indicating prognosis for a subject diagnosed with bladder cancer, the method comprising: receiving an activity of a Notch cellular signaling pathway determined in an urothelial cell sample obtained from the subject; comparing the received activity to one or more reference activities, and determining the prognostic score, wherein the prognostic score is defined such that the prognosis is worse the higher the activity is.

This aspect of the present invention resides on the innovation of the inventors that the activity of the Notch cellular signaling pathway serves as a prognostic marker for bladder cancer, and more specifically on the finding that prognosis is inversely correlated with the activity, meaning that a (relatively) high activity is indicative of a (relatively) poor prognosis and a (relatively) low activity is indicative of a (relatively) favorable prognosis. Thereby, the activity of the Notch cellular signaling pathway in bladder cancer cells obtained from the subject can be used as a surrogate marker for disease progression in subjects having bladder cancer. As an advantage, this aspect of the present invention facilitates identification of a subject that is expected to have a poor prognosis at an early stage of the disease and therefore enables better treatment options than existing methods. This aspect of the present invention may aid in selection of individuals for close CT monitoring and secondary chemoprevention.

The term "subject", as used herein, refers to any living being. In some embodiments, the subject is an animal, preferably a mammal. In certain embodiments, the subject is a human being, such as a medical subject. A "subject diagnosed with bladder cancer", as used herein, refers, without limitation, to a subject, which has bladder cancer, is suspected to have bladder cancer or has been diagnosed to have bladder cancer, irrespective whether or not the diagnosis turns out to be incorrect, or whether or not the subject has meanwhile recovered. In specific embodiments, the subject may not receive or may not have received chemotherapy. For instance, the subject may not have received chemotherapy within the last 12 months, preferably 9 months, more preferably 6 months and most preferably 3, 2 or 1 month.

An "urothelial cell sample" to be used in accordance with the present invention can be an extracted sample, that is, a sample that has been extracted from the subject. Examples of the sample include, but are not limited to epithelial cells, tissue and/or body fluid containing urothelial cells from the subject. The respective sample can, for example, be obtained from the subject's bladder by cystoscopy and/or isolated from the urine. Advantageously, the method of the present invention can be used as a standalone assay or in combination with existing diagnostic methods, such as voided urine cytology and/or cystoscopic examination of the bladder.

The term "sample", as used herein, also encompasses the case where e.g. cells, tissue and/or body fluid have been taken from the subject and, e.g., have been put on a microscope slide or fixative, and where for performing the claimed method a portion of this sample is extracted, e.g., by means of Laser Capture Microdissection (LCM), or by punching, or by scraping off the cells of interest from the slide, or by fluorescence-activated cell sorting techniques. In addition, the term "sample", as used herein, also encompasses the case where e.g. cells, tissue and/or body fluid have been taken from the subject and have been put on a microscope slide, and the claimed method is performed on the slide.

The terms "pathway", "signal transduction pathway", "signaling pathway" and "cellular signaling pathway" are used interchangeably herein. The Notch cellular signaling pathway as understood herein is defined as a cellular signaling pathway that comprises a protein receptor from the Notch family, and a family of (cell-bound) ligands (DSL family) which induce cleavage of the bound receptor, upon which the cleaved intracellular fragment moves to the nucleus, where it forms, together with other proteins, an active Notch transcription factor complex which binds and transactivates a set of Notch target genes. Preferably, the protein receptor is a NOTCH2 receptor.

The activity of the Notch cellular signaling pathway is to be understood to be a measure reflecting the degree of activation of said pathway. It may refer, or it may be defined to be related, in particular to be proportional, to the activity of a Notch signaling pathway associated transcription factor (TF) element in the sample, the Notch TF element controlling transcription of Notch target genes, in driving the Notch target genes to expression, i.e., the speed by which the Notch target genes are transcribed, e.g. in terms of high activity (i.e. high speed) or low activity (i.e. low speed), or other dimensions, such as levels, values or the like related to such activity (e.g. speed). Accordingly, the activity of the Notch cellular signaling pathway associated TF element can be directly used as a measure reflecting the degree of activation of the Notch pathway, and thus, for the purposes of the present invention, the term "activity", as used herein, is also meant to refer to an activity level of the Notch signaling pathway associated transcription factor TF element. As will be appreciated by a person skilled in the art, the form or unit of the activity of the Notch cellular signaling pathway is not particularly limited, as long as its relation to the form and unit of the reference activity is known. Conveniently, the received activity has the same form and unit as the reference activity.

The term "reference activity" as used herein denotes a predetermined activity of the Notch cellular signaling pathway, which activity is defined to be associated with a predetermined prognosis. Reference activities, for which a predetermined prognosis can be retrospectively defined by reviewing reported disease progression following sampling, can be conveniently deduced from existing, e.g. publicly available, sample data in the same way as for the "activity". Based on the reference activity and the associated predetermined prognosis, a reference prognostic score may be defined in place of and reflecting the respective predetermined prognosis. Principally, it is sufficient to make a comparison of the received activity to one reference activity, in which case it can be determined whether the received activity is above, substantially identical or below the reference activity so as to allow a prognosis as compared to the reference, i.e. worse, same or better prognosis as compared to the reference. In order to allow a more detailed prognosis, it is preferred to consider more than one reference activity. For example, a plurality of reference activities associated with a plurality of prognoses may be used to develop a mathematical model calibrated with said plurality of references, which mathematical model is compared to the received activity so as to determine a prognosis. Preferably, one or more reference activities are provided that are known to be associated with one or more disease stages, e.g., non-muscle invasive bladder cancer, muscle-invasive bladder cancer, metastasizing bladder cancer, and the like, associated with one or more prognoses.

By comparing the Notch pathway activity of the subject to be prognosticated with the one or more reference activities, a prognostic score can be determined. The prognostic score is a measure that reflects and preferably allows drawing qualitative, semi-quantitative or quantitative conclusions towards the subject's prognosis. Hereto forth, different scores may be assigned to different (ranges of) pathway activities. For example, a first score may be defined so as to be assigned to an upper pathway activity (range) above a predetermined reference activity, wherein the predetermined reference activity serves as a threshold, above which a poor prognosis is to be expected (relative to a prognosis associated with the reference activity). A second score may further be defined that is assigned to a lower pathway activity (range) below said predetermined reference activity, the second score indicating a subject having a favorable prognosis (relative to a prognosis associated with the reference activity). The upper and/or lower pathway activity (ranges) may be further divided into sub-ranges, to which respective scores are assigned so as to allow a semi-quantitative or quantitative prediction on the prognosis to be expected. By convention, the prognostic score is defined such that the prognosis is worse the higher the activity is. The prognostic score may thereby be translated into a prognosis.

Preferably, the prognosis is indicative of a risk of having or acquiring muscle invasive and/or a risk of having or acquiring metastatic bladder cancer, and/or time to metastasis, and/or survival rate, and/or time of progression of bladder cancer, and/or risk of relapse of bladder cancer. This means a (relatively) high Notch pathway activity is indicative of a (relatively) high risk of having or acquiring muscle invasive and/or high risk of having or acquiring metastatic bladder cancer, and/or little time to metastasis, and/or low survival rate, and/or fast progression of bladder cancer, and/or high risk of relapse of bladder cancer, and vice versa.

Accordingly, in a preferred embodiment of the first aspect, the prognostic score is defined such that an increasing activity is associated with an increasing risk of having or acquiring muscle invasive and/or an increasing risk of metastatic bladder cancer and/or less time to metastasis and/or lower survival rate and/or faster progression of bladder cancer and/or an increased risk of relapse of bladder cancer. The present invention is therefore capable of providing information on the stage of the disease. According to a specific embodiment, a muscle-invasive bladder cancer can be distinguished from a non-muscle-invasive bladder cancer. In this embodiment, the prognostic score is defined such that a higher activity is more associated with muscle invasive bladder cancer than with non-muscle invasive bladder.

In a preferred embodiment of the first aspect of the invention of the invention, the step of comparing the received activity to one or more reference activities of the Notch cellular signaling pathway comprises evaluating the received activity against a calibrated mathematical model relating the one or more reference activities to respective one or more reference prognostic scores. The term "calibrated mathematical model" is to be understood as a model having an input (here, the received Notch pathway activity) and an output (here, the prognostic score) that is adapted based on reference or calibration data, i.e., data for which both the input values and the output values are known, so that the model yields the known output values given the input values.

In accordance with a second aspect of the present invention, the above problem is solved by a method of determining a response score indicating responsiveness of a subject diagnosed with bladder cancer to treatment with an inhibitor of the Notch cellular signaling pathway, the method comprising: receiving an activity of a Notch cellular signaling pathway determined in an urothelial cell sample obtained from the subject; comparing the received activity to one or more reference activities of the Notch cellular signaling pathway, and determining the response score, wherein the response score is defined such that the activity is positively correlated with responsiveness.

The second aspect of the invention resides on the finding of the inventors that increased activity of the Notch cellular signaling pathway drives disease progression of bladder cancer towards a more lethal metastatic form. This means, the activity of the Notch cellular signaling pathway does not only serve as a prognostic marker but can also be used as a marker that is indicative for the likelihood that a bladder cancer is responsive to treatment with an inhibitor of the Notch cellular signaling pathway. This preferably allows improving the possibilities of characterizing patients that have a disease, which is at least partially driven by an abnormal activity of the Notch cellular signaling pathway, and that are therefore likely to respond to inhibitors of the Notch cellular signaling pathway.

By comparing the Notch pathway activity of the subject with the one or more reference activities, a response score can be determined. The response score is a measure that allows drawing qualitative, semi-quantitative or quantitative conclusions towards the subject's responsiveness towards treatment with an inhibitor of the Notch cellular signaling pathway. Similar to the first aspect, different scores may be assigned to different (ranges of) pathway activities. For example, a first score may be defined so as to be assigned to an upper pathway activity (range) above a predetermined reference activity, wherein the predetermined reference activity serves as a threshold, above which a good responsiveness is to be expected (relative to a responsiveness associated with the reference activity). A second score may further be defined that is assigned to a lower pathway activity (range) below said predetermined reference activity, the second score indicating a subject having a bad responsiveness (relative to a responsiveness associated with the reference activity). The upper and/or lower pathway activity (ranges) may be further divided into sub-ranges, to which respective scores are assigned so as to allow a semi-quantitative or quantitative prediction on the responsiveness to be expected. By convention, the response score is defined such that the activity is positively correlated with the responsiveness, i.e. the responsiveness is better the higher the activity is, and vice versa.

The reference activity as used in the second aspect may be the same as the reference activity of the first aspect, except that it is defined to be associated with a predetermined responsiveness (reflected by a reference prognostic score) instead of a predetermined prognosis. However, since the Notch pathway activity is assumed to be causally linked to both prognosis and responsiveness, the predetermined reference prognosis may equally serve as indicator of the predetermined reference response, wherein the prognostic score and response score are defined such that prognosis and response are better the higher the Notch pathway activity is, and vice versa.

Suitable Notch pathway inhibitors are known to a person skilled in the art and include, without limitation, blocking antibodies directed against Notch receptors, blocking antibodies against Notch ligands, decoys, gamma-secretase inhibitors, and so forth. Specific examples include DAPT, PF-03084014, MK-0752, RO-4929097, LY450139, BMS-708163, LY3039478, IMR-1, Dibenzazepine, LY411575, FLI-06. Preferably, the Notch pathway inhibitor is a NOTCH2 receptor inhibitor. A NOTCH2 receptor inhibitor, as understood herein, is an inhibitor that inhibits a signaling cascade initiated by binding to the NOTCH2 receptor. Suitable inhibitors in this regard are in particular inhibitors that prevent the ligand from binding to the NOTCH2 receptor or inhibit the signaling cascade downstream of the NOTCH2 receptor, for instance an inhibitor which prevents formation of the Notch TF element or target genes directly controlled by the Notch TF element.

With respect to further explanations, definitions and further details regarding terminology used for describing the second aspect, reference is made to the first aspect, which description applies correspondingly, unless otherwise stated herein or dictated by context.

In a preferred embodiment of the second aspect of the invention, the method further comprises stratifying the subject between at least two groups of responders and non-responders. As an advantage, this embodiment facilitates identification of responders and non-responders so as to provide a potential (secondary) treatment specifically for those that are likely to benefit from treatment with an inhibitor of the Notch cellular signaling pathway.

In a preferred embodiment of the first and second aspect of the invention, the method further comprises providing the determined prognostic score and the determined response score, respectively. For instance, the respective score may be reported to a qualified person so as to allow the person to make a prognosis, to predict responsiveness and/or prescribe a medicament such as a Notch pathway inhibitor, preferably a NOTCH2 receptor inhibitor.

In a preferred embodiment of the second aspect of the invention, the step of comparing the received activity to one or more reference activities of the Notch cellular signaling pathway comprises evaluating the received activity against a calibrated mathematical model relating the one or more reference activities to respective one or more reference response scores.

In a preferred embodiment of the first and second aspect of the invention, the activity of the Notch cellular signaling pathway is defined to be related to an activity level of a Notch cellular signaling pathway associated transcription factor (TF) element.

Herein, the term "Notch transcription factor element" or "Notch TF element" or "TF element" is defined as a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-Jκ, Su(H) and LAG-1), which is capable of binding to specific DNA sequences, and preferably one co-activator protein from the mastermind-like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

The activity level of a TF element denotes the level of activity of the TF element regarding transcription of its target genes. Thus, the activity of the Notch cellular signaling pathway can be determined based on a measurement of the signaling output of the Notch cellular signaling pathway, which is - amongst others - the transcription of the target genes, which is controlled by a Notch transcription factor (TF) element that is controlled by the Notch cellular signaling pathway.

In accordance with said relationship, a preferred embodiment of the first and second aspect of the invention is a method (as described herein), wherein the activity of the Notch cellular signaling pathway is inferred or inferable by a method comprising: receiving expression levels of three or more target genes of the Notch cellular signaling pathway, determining an activity level of a Notch cellular signaling pathway associated TF element, the TF element controlling transcription of the three or more target genes, the determining being based on evaluating a calibrated mathematical pathway model relating the expression levels of the three or more target genes to the activity level of the TF element, and inferring the activity of the Notch cellular signaling pathway based on the determined activity level of the TF element.

The term "target gene", as used herein, means a gene whose transcription is directly or indirectly controlled by the Notch TF element. The "target gene" may be a "direct target gene" and/or an "indirect target gene".

In an embodiment the expression levels of the target genes are determined using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

In a preferred embodiment of the first and second aspect of the invention, the three or more, for example, three, four, five, six, seven, eight, nine, ten, eleven, twelve, 13, 14, 15, 16, 17, 18, 19, 20, 21, 22, 23, 24, 25 or more, Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC. Preferably, two or more, for example, three, four, five, six, seven or more, Notch target genes are selected from the group consisting of: DTX1, HES1, HES4, HES5, HEY2, MYC, NRARP, and PTCRA, and one or more, for example, two, three, four, five, six, seven, eight, nine, ten, eleven, twelve, 13, 14, 15, 16, 17 or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC. This embodiment makes it possible to determine the activity of the Notch cellular signaling pathway by (i) determining an activity level of a Notch TF element in the sample of the subject, wherein the determining is based on evaluating a calibrated mathematical model relating the expression levels of three or more target genes of the Notch cellular signaling pathway, the transcription of which is controlled by the Notch TF element, to the activity level of the Notch TF element, and by (ii) inferring the activity of the Notch cellular signaling pathway in the subject based on the determined activity level of the Notch TF element in the sample of the subject. This preferably allows improving the possibilities of developing a prognosis for patients being diagnosed with bladder cancer, which is at least partially driven by an increased activity of the Notch cellular signaling pathway, and that are therefore likely to respond to inhibitors of the Notch cellular signaling pathway. In particular embodiments, treatment determination can be based on a specific Notch cellular signaling pathway activity. In a particular embodiment, the Notch cellular signaling status can be set at a cutoff value of odds of the Notch cellular signaling pathway being active of, for example, 10:1, 5:1, 4:1, 2:1, 1:1, 1:2, 1:4, 1:5, or 1:10.

The calibrated mathematical pathway model is preferably a centroid or a linear model, or a Bayesian network model based on conditional probabilities. For example, the calibrated mathematical pathway model may be a probabilistic model, preferably a Bayesian network model, based on conditional probabilities relating the activity level of the Notch TF element and the expression levels of the three or more Notch target genes, or the calibrated mathematical pathway model may be based on one or more linear combination(s) of the expression levels of the three or more Notch target genes. In particular, the inferring of the activity of the Notch cellular signaling pathway may be performed as disclosed in the published international patent application WO 2013/011479 A2 ("Assessment of cellular signaling pathway activity using probabilistic modeling of target gene expression") or as described in the published international patent application WO 2014/102668 A2 ("Assessment of cellular signaling pathway activity using linear combination(s) of target gene expressions"), the contents of which are herewith incorporated in their entirety. Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

Particularly preferred is a method (as described herein), wherein the three or more (e.g., four, five, six, seven, eight, nine, ten, eleven, twelve, 13, 14, 15, 16, 17 or more) Notch target genes are selected from the group consisting of: CD44, DTX1, EPHB3, HES1, HES4, HES5, HES7, HEY1, HEY2, HEYL, MYC, NFKB2, NOX1, NRARP, PBX1, PIN1, PLXND1, and SOX9, preferably, wherein two or more, for example, three, four, five, six or more, Notch target genes are selected from the group consisting of: DTX1, HES1, HES4, HES5, HEY2, MYC, and NRARP, and one or more, for example, three, four, five, six, seven, eight, nine, ten or more, Notch target genes are selected from the group consisting of: CD44, EPHB3, HES7, HEY1, HEYL, NFKB2, NOX1, PBX1, PIN1, PLXND1, and SOX9. Particularly preferred is a method (as described herein), wherein the three or more (e.g., four, five, six, seven, eight, nine, ten, eleven or more) Notch target genes are selected from the group consisting of: DTX1, EPHB3, HES1, HES4, HES5, HEY2, MYC, NFKB2, NRARP, PIN1, PLXND1, and SOX9, preferably, wherein two or more, for example, three, four, five, six or more, Notch target genes are selected from the group consisting of: DTX1, HES1, HES4, HES5, HEY2, MYC, and NRARP, and one or more, for example, three, four or more, Notch target genes are selected from the group consisting of: EPHB3, NFKB2, PIN1, PLXND1, and SOX9.

Further and/or preferred Notch target genes or Notch target gene combinations are described in EP17194288.1 (filed on Oct 2, 2017; titled "Assessment of Notch cellular signaling pathway activity using mathematical modelling of target gene expression"), which is incorporated herein in its entirety, in particular for the purpose of disclosing Notch target genes and gene combinations, in particular those shown in Table 1, Table 2 and/or Table 3, along with respective sequence listings for the target genes disclosed herein as well as in said reference. Furthermore, reference is made to the example section, which describes not only preferred Notch target genes but also a specific way of determining Notch pathway activity, both of which are incorporated herein.

In a further preferred embodiment of the first and second aspect of the invention, the method is computer-implemented to be performed by a digital processing device.

In accordance with another disclosed aspect, an apparatus for determining a prognostic score indicating prognosis for a subject diagnosed with bladder cancer or determining a response score indicating responsiveness of a subject diagnosed with bladder cancer to treatment with an inhibitor of the Notch cellular signaling pathway comprises a digital processor configured to perform the method of the first or second aspect of the present invention as described herein.

In accordance with another disclosed aspect, a non-transitory storage medium for determining a prognostic score indicating prognosis for a subject diagnosed with bladder cancer or determining a response score indicating responsiveness of a subject diagnosed with bladder cancer to treatment with an inhibitor of the Notch cellular signaling pathway stores instructions that are executable by a digital processing device to perform the method of the first or second aspect of the present invention as described herein. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with another disclosed aspect, a computer program for determining a prognostic score indicating prognosis for a subject diagnosed with bladder cancer or determining a response score indicating responsiveness of a subject diagnosed with bladder cancer to treatment with an inhibitor of the Notch cellular signaling pathway comprises program code means for causing a digital processing device to perform the method of the first or second aspect of the present invention as described herein, when the computer program is run on the digital processing device. The digital processing device may be a handheld device (e.g., a personal data assistant or smartphone), a notebook computer, a desktop computer, a tablet computer or device, a remote network server, or so forth.

In accordance with another disclosed aspect, a kit for determining a prognostic score indicating prognosis for a subject diagnosed with bladder cancer or determining a response score indicating responsiveness of a subject diagnosed with bladder cancer to treatment with an inhibitor of the Notch cellular signaling pathway comprises: components for determining the expression levels of at least three target genes (as disclosed herein) of a Notch cellular signaling pathway, and the apparatus as disclosed herein, the non-transitory storage medium as disclosed herein, or the computer program as disclosed herein.

The set of target genes which are found to best indicate the activity of the Notch cellular signaling pathway, based on microarray/RNA sequencing based investigation using, e.g., the Bayesian model or the (pseudo-)linear model, can be translated into for example a multiplex quantitative PCR assay or dedicated microarray biochips to be performed on a sample of a subject. A selection of the gene sequence as described herein can be used to select for example a primer-probe set for RT-PCR or oligonucleotides for microarray development.

Preferably, the three or more (e.g., four, five, six, seven, eight, nine, ten, eleven, twelve, 13, 14, 15, 16, 17 or more) Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC. Preferably, two or more, for example, three, four, five, six, seven or more, Notch target genes are selected from the group consisting of: DTX1, HES1, HES4, HES5, HEY2, MYC, NRARP, and PTCRA, and one or more, for example, three, four, five, six, seven, eight, nine, ten, eleven, twelve, 13, 14, 15, 16, 17 or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

Particularly preferred is a kit (as described herein), wherein the three or more (e.g., four, five, six, seven, eight, nine, ten, eleven, twelve, 13, 14, 15, 16, 17 or more) Notch target genes are selected from the group consisting of: CD44, DTX1, EPHB3, HES1, HES4, HES5, HES7, HEY1, HEY2, HEYL, MYC, NFKB2, NOX1, NRARP, PBX1, PIN1, PLXND1, and SOX9, preferably, wherein two or more, for example, three, four, five, six or more, Notch target genes are selected from the group consisting of: DTX1, HES1, HES4, HES5, HEY2, MYC, and NRARP, and one or more, for example, three, four, five, six, seven, eight, nine, ten or more, Notch target genes are selected from the group consisting of: CD44, EPHB3, HES7, HEY1, HEYL, NFKB2, NOX1, PBX1, PIN1, PLXND1, and SOX9.

Particularly preferred is a kit (as described herein), wherein the three or more Notch target genes (e.g., four, five, six, seven, eight, nine, ten, eleven or more) are selected from the group consisting of: DTX1, EPHB3, HES1, HES4, HES5, HEY2, MYC, NFKB2, NRARP, PIN1, PLXND1, and SOX9, preferably, wherein two or more, for example, three, four, five, six or more, Notch target genes are selected from the group consisting of: DTX1, HES1, HES4, HES5, HEY2, MYC, and NRARP, and one or more, for example, three, four or more, Notch target genes are selected from the group consisting of: EPHB3, NFKB2, PIN1, PLXND1, and SOX9.

The one or more components or means for measuring the expression levels of the three or more Notch target genes can be selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers. Preferably, the kit is selected from the group consisting of qPCR, multiple qPCR, multiplexed qPCR, ddPCR, RNAseq, RNA expression array and mass spectrometry. In an embodiment, the kit includes a set of labeled probes directed to a portion of an mRNA or cDNA sequence of the three or more Notch target genes as described herein. In an embodiment, the kit includes a set of primers and probes directed to a portion of an mRNA or cDNA sequence of the three or more Notch target genes. In an embodiment, the labeled probes are contained in a standardized 96-well plate. In an embodiment, the kit further includes primers or probes directed to a set of reference genes. Such reference genes can be, for example, constitutively expressed genes useful in normalizing or standardizing expression levels of the target gene expression levels described herein.

In an embodiment, the kit for measuring the expression levels of three or more, for example, three, four, five, six, seven, eight, nine, ten, eleven, twelve or more, target genes of the Notch cellular signaling pathway in a sample of a subject comprises: polymerase chain reaction primers directed to the three or more Notch target genes, probes directed to the three or more Notch target genes.

Another aspect of the present invention pertains to use of a kit comprising components for determining the expression levels of at least three target genes of a Notch cellular signaling pathway for determining a prognostic score indicating prognosis for a subject diagnosed with bladder cancer or determining a response score indicating responsiveness of a subject diagnosed with bladder cancer to treatment with an inhibitor of the Notch cellular signaling pathway. Preferably, the determining of the expression levels of at least three target genes of a Notch cellular signaling pathway for determining a prognostic score indicating prognosis for a subject diagnosed with bladder cancer is in accordance with the first aspect of the present invention as described herein. Preferably, the determining of a response score indicating responsiveness of a subject diagnosed with bladder cancer to treatment with an inhibitor of the Notch cellular signaling pathway is in accordance with the second aspect of the present invention as described herein.

Further advantages will be apparent to those of ordinary skill in the art upon reading and understanding the attached figures, the following description and, in particular, upon reading the detailed examples provided herein below.

It shall be understood that the methods of claims 1 and 4, the apparatus of claim 11, the non-transitory storage medium of claim 12, the computer program of claim 13, the kit of claim 14, and the use of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows Notch cellular signaling pathway activity predictions as log2odds of the trained exemplary Bayesian network model using the 18 target genes from Table 2 of EP17194288.1 on sample data from dataset GSE31684, wherein sample data from subjects having a muscle invasive (MI) bladder cancer are shown on the left and sample data from subjects having non-MI bladder cancer are shown on the right. Calibration and validation was done as described in EP17194288.1,
Fig. 2 shows the Notch cellular signaling pathway activity predictions of Fig. 1, wherein sample data from subjects of Cluster 1 are shown on the left and sample data from subjects of Cluster 2 (poor prognosis) are shown on the right,
Fig. 3 shows the Notch cellular signaling pathway activity predictions Fig. 1, wherein sample data from subjects having no metastasis are shown on the left and sample data from subjects having distant metastasis are shown on the right,
Fig. 4 shows a Kaplan Meier curve indicating recurrence free survival probabilities over time (in months) for patients with a Notch-inactive tumor (A) and patients having a Notch-active tumor (B) determined from the Notch cellular signaling pathway activity predictions of Fig. 1. Patients that received chemotherapy have been excluded in order to eliminate any potential influence of the chemotherapy on the results. For instance, it is possible that chemotherapy interferes with pathway activities,
Fig. 5 shows a flow chart exemplarily illustrating a process for inferring activity of the Notch cellular signaling pathway in a subject based on expression levels of target genes of the Notch cellular signaling pathway measured in a sample of a subject,
Fig. 6 shows a flow chart exemplarily illustrating a process for determining a prognostic score indicating prognosis based on activity of a Notch cellular signaling pathway,
Fig. 7 shows a flow chart exemplarily illustrating a process for determining a response score indicating responsiveness to treatment with an inhibitor of the Notch cellular signaling pathway prognosis based on activity of a Notch cellular signaling pathway,
Fig. 8 shows a correlation plot between the Notch pathway activity (y-axis) inferred from samples of bladder cancer patients and expression levels of different NOTCH receptors (x-axis). For the NOTCH1, NOTCH2 and NOTCH3 receptors, more than one plot is shown because there are data available from more than one probeset. The probesets were as follows (from top left to bottom right): NOTCH1.218902_at, NOTCH1.223508_at, NOTCH2.202443_x_at, NOTCH2.202445_s_at, NOTCH2.210756_s_at, NOTCH2.212377_s_at, NOTCH3.203237_s_at, NOTCH3.203238_s_at, NOTCH4.205247_at, and
Fig. 9 shows a Kaplan Meier curve indicating recurrence free survival probabilities over time (in months) for patients with increased NOTCH2 receptor expression (B) and patients having no increased NOTCH2 receptor expression (A).

### DETAILED DESCRIPTION OF EMBODIMENTS

The invention relates to a method of determining a prognostic score indicating prognosis for a subject diagnosed with bladder cancer. The method comprises receiving an activity of a Notch cellular signaling pathway determined in an urothelial cell sample obtained from the subject. The received activity is compared to one or more reference activities. The prognostic score is determined, wherein the prognostic score is defined such that the prognosis is worse the higher the activity is. The method advantageously allows to draw prognosis with respect to the risk of having or acquiring muscle invasive or metastatic bladder cancer, or with respect to time to metastasis, survival rate or progression of bladder cancer, or risk of relapse of bladder cancer.

The following examples merely illustrate particularly preferred methods and selected aspects in connection therewith and are not to be construed as limiting the scope of the present invention.

A novel method has been described to quantitatively measure activity of signal transduction pathways (estrogen and androgen receptor, PI3K, Hedgehog, Wnt, TGFβ and NFkB) in a tissue sample based on Bayesian models that infer a pathway activity score from transcription factor target gene mRNA levels (Verhaegh et al., Cancer Res., vol. 74, no. 11, pp. 2936-2945, Jun. 2014; van Ooijen et al., Am. J. Pathol., vol. 188, no. 9, pp. 1956-1972, Sep. 2018; van de Stolpe, Holtzer, van Ooijen, de Inda, and Verhaegh, Sci Rep, vol. 9, no. 1, p. 1603, Feb. 2019). In particular, a method for inferring activity of a Notch cellular signaling pathway has been developed, validated and described in EP17194288.1 (*supra*), which method is incorporated herein by reference. In this context, particular reference is made to the section "Detailed Description of Embodiments" and more specifically to Example 1 ("Mathematical model construction"), Example 2 ("Selection of target genes") and Example 3 ("Training and using the mathematical model"), which provide particular modes of implementing the method.

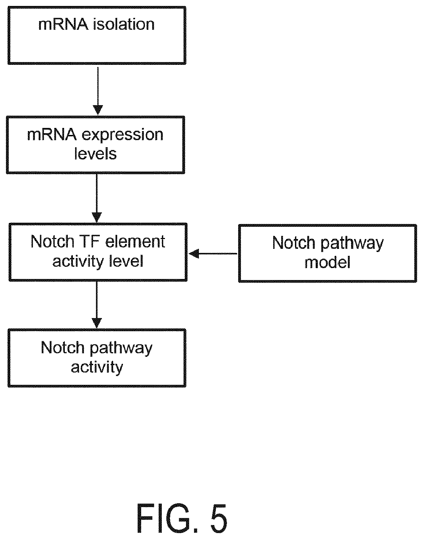

A flowchart exemplarily illustrating a process for inferring the activity of Notch cellular signaling from a sample isolated from a subject is shown in Fig. 5. First, the mRNA from a sample is isolated. Second, the mRNA expression levels of a unique set of at least three or more Notch target genes, as described herein, are measured using methods for measuring gene expression that are known in the art. Next, an activity level of a Notch transcription factor (TF) element is determined using a calibrated mathematical pathway model relating the expression levels of the three or more Notch target genes to the activity level of the Notch TF element. Finally, the activity of the Notch cellular signaling pathway in the subject is inferred based on the determined activity level of the Notch TF element in the sample of the subject.

Using this method, the activity of the Notch cellular signaling pathway in bladder cancer cell samples was inferred by the inventors of the present invention in order to investigate the role of said signaling pathways in bladder cancer.

Pathway analysis was performed on publicly available AffymetrixU133 Plus2.0 data (GSE31684) of microdissected cancer tissue samples from 93 bladder cancer patients with muscle-invasive (MI) or non-MI disease who underwent radical cystectomy without systemic therapy. The patients had been divided into two known RNA-expression-based clusters, Cluster 2 is characterized by bad prognosis (Riester et al., Clin. Cancer Res., vol. 18, no. 5, pp. 1323-1333, Mar. 2012). Pathway activities were calculated as log2odds, as extensively described in EP17194288.1 (*supra*). Subsequently, two-sided Wilcoxon signed-rank statistical tests were performed. For the Kaplan Meier curve a predefined Notch pathway activity threshold was used.

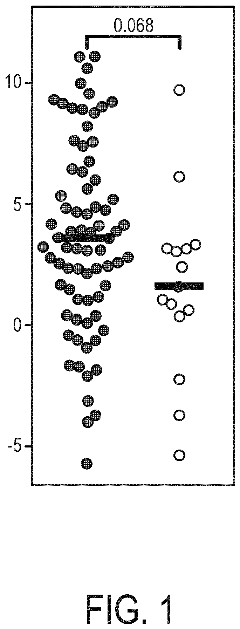

In the diagrams shown in Figs 1 to 3, the horizontal axis indicates the odds (on a log2 scale) that the Notch TF element is "present" resp. "absent", which corresponds to the Notch cellular signaling pathway being active resp. inactive, as predicted by the trained exemplary Bayesian network model. Different patient groups are depicted on separate columns. For each patient group (column), the mean is indicated as horizontal bar.

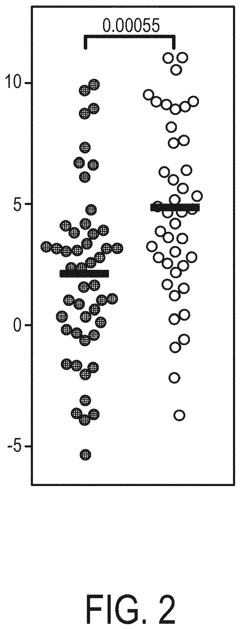

As shown in Fig. 1, the Notch pathway activity was found to be higher in muscle invasive (MI) (depicted in the graph on the left) versus non-MI cancer (p=0.068). As shown in Fig. 2, the Notch pathway activity was found to be higher in Cluster 2 patients (p=0.0005; depicted in the graph on the right) as compared to Cluster 1 patients.

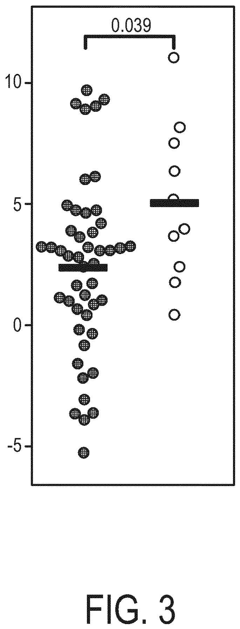

As shown in Fig. 3, the Notch pathway activity was found to be higher in patients with distant metastasis (p=0.039 depicted in the graph on the right) as compared to those that had no metastasis.

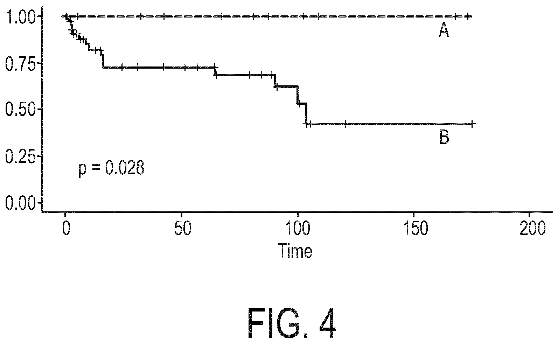

Fig. 4 shows a Kaplan Meier curve indicating recurrence free survival probabilities over time for patients (receiving no chemotherapy) having a Notch-inactive tumor (A, log2odds < 0) and patients (receiving no chemotherapy) having a Notch-active tumor (B, log2odds >= 0), as determined from the Notch pathway activity predictions (upper graph). Patients with a Notch-inactive tumor were found to have a survival probability of 100 %, whereas Notch-active tumors were found to indicate patients, whose survival probability drops over time. Despite low patient numbers a Kaplan Meier curve showed that patients with a Notch-inactive tumor did not get metastasis or local recurrence (n=11), in contrast to Notch-active tumors (n=46, p=0.028), while the Notch pathway activity score was inversely correlated with time to metastasis (Hazard-ratio, 1.192051, p=0.039) (Fig. 4).

From the results, it can be concluded that the Notch pathway has a tumor and/or metastasis-driving role of in bladder cancer, associated with a bad prognosis. Measuring of Notch pathway activity may thus be of value to identify patients at high risk for metastasis and potentially stratify patients for (complementary) Notch-inhibitory treatment.

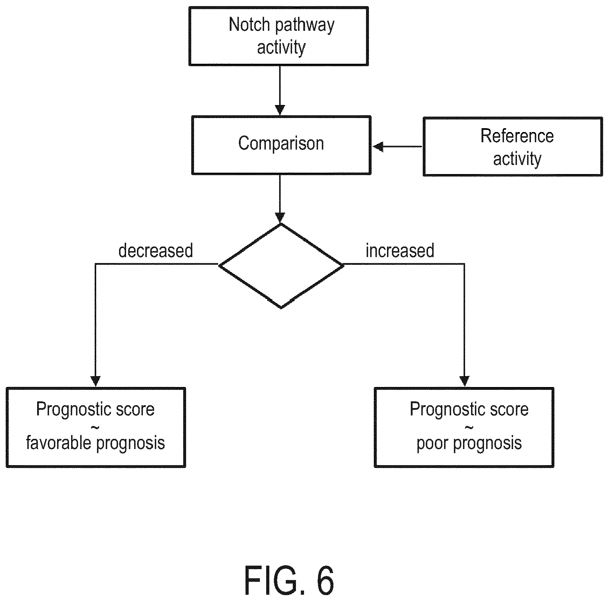

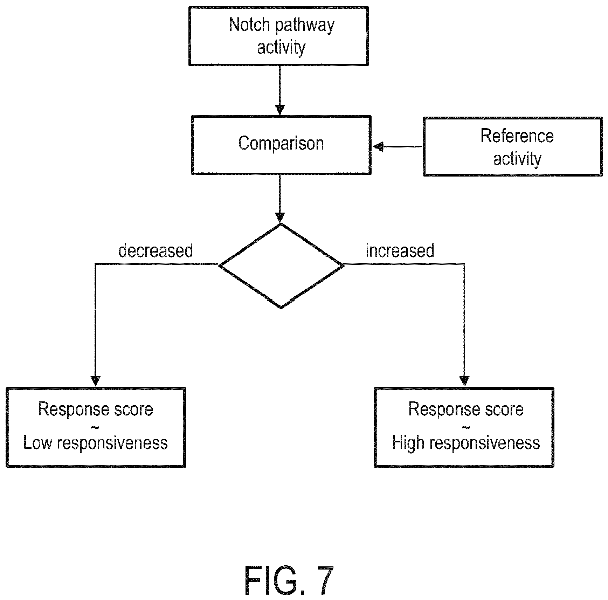

A flowchart exemplarily illustrating a process for determining a prognostic score and a response score based on the activity of Notch cellular signaling is shown in Figs. 6 and 7, respectively. First, the activity of the Notch cellular signaling pathway determined in a sample of a subject diagnosed with bladder cancer is received. For example, the activity may be determined according to the process illustrated in Fig. 5. The activity is input to a step of comparison to one or more reference activities for which respective prognoses / prognostic scores and respective responses / response scores, respectively, are known, as disclosed herein. Reference data can be experimentally determined or derived from expert knowledge and stored in the form of a threshold value, calibration curve and/or a (more complex) calibrated model for later use. Such later use of a threshold value, calibration curve and/or a calibrated model is understood to involve a use of the one or more reference activities in a step of comparing the inferred pathway activity with one or more reference activities. Output of the comparison step is a prognostic score and response score, respectively, which are defined to be correlated with the Notch pathway activity such that the prognosis is worse the higher the activity is (prognostic score), respectively, the responsiveness is higher the higher the activity is (response score).

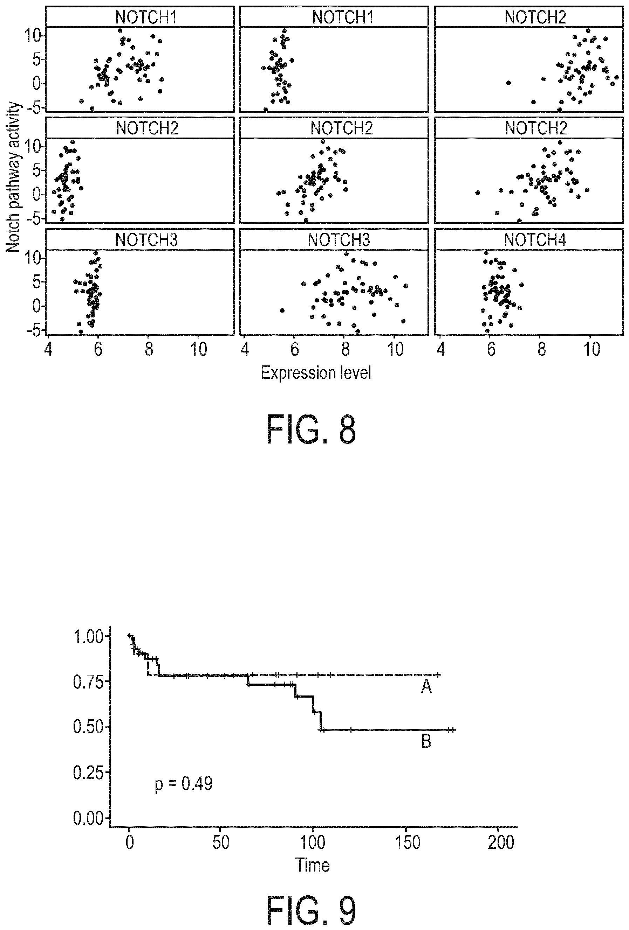

Referring to Fig. 8, the expression levels of different Notch receptors has been correlated to the Notch pathway activity inferred with the model as disclosed herein for bladder cancer patients. It was observed that the correlation is highest for the NOTCH2 receptor, while for the other Notch receptors there is no significant correlation (NOTCH3 and NOTCH4) or a lower correlation (NOTCH1). These results clearly suggest that the NOTCH2 receptor is tumor-promoting (i.e. oncogenic), whereas the NOTCH3 and NOTCH4 receptors are not and the NOTCH1 receptor is less oncogenic. To the knowledge of the inventors, this is the first time of proof from clinical data that the NOTCH2 receptor is the tumor-driving receptor in Notch signaling. The results are summarized in the following table:

| Probeset | Correlation coefficient | p-value |
|---|---|---|
| NOTCH1.218902_at | 0.34 | 0.001 |
| NOTCH1.223508_ at | 0.05 | 0.72 |
| NOTCH2.2Q2443_x_at | 0.30* | 0.02 |
| NOTCH2.202445_s_at | 0.31* | 0.02 |
| NOTCH2.210756_s_at | 0.56 | 6e-06 |
| NOTCH2.212377_s_at | 0.40 | 0.002 |
| NOTCH3.203237_s_at | 0.19 | 0.17 |
| NOTCH3.203238_s_at | 0.15 | 0.28 |
| NOTCH4.205247_at | -0.08 | 0.55 |

| | | |
|---|---|---|
| *Comment: Due to a probeset sensitivity < 0.7, the probesets indicated with "*" were neglected from further studies. | | |

While Fig. 4 show Kaplan-Meier curves based on Notch pathway activity, Fig. 9 shows a corresponding plot that has been determined based on NOTCH2 expression levels using probeset 210756_s_at. The Kaplan Meier curve indicates recurrence free survival probabilities over time for patients (receiving no chemotherapy) having increased expression of the NOTCH2 receptor (B) and patients (receiving no chemotherapy) not having increased expression of the NOTCH2 receptor (A), as determined from the NOTCH2 expression results (Fig. 8). The threshold was chosen between High and Low such that the resulting groups sizes are the same as for the Notch pathway Kaplan-Meier plot shown in Fig. 4. Patients with no increase in expression of the NOTCH2 receptor were found to have a survival probability of about 75 %, whereas patients having increased NOTCH2 receptor-expression were found to have survival probability that drops down to about 50 % over time (n=46, log rank p=0.49). Notably, NOTCH2 expression levels were not significantly correlated with survival probabilities over time (p=0.49). This is in contrast to the Notch pathway activity, which in fact showed a statistically significant relationship (p=0.028), highlighting the predictability and reliability of the method of the present invention.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the determination of the prognostic or response score, the determination of Notch pathway activity, et cetera, performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A method of determining a prognostic score indicating prognosis for a subject diagnosed with bladder cancer, the method comprising:
- receiving an activity of a Notch cellular signaling pathway determined in an urothelial cell sample obtained from the subject;
- comparing the received activity to one or more reference activities, and
- determining the prognostic score,
wherein the prognostic score is defined such that the prognosis is worse the higher the activity is.

2. The method of claim 1, wherein the prognostic score is defined such that an increasing activity is associated with an increasing risk of having or acquiring muscle invasive and/or an increasing risk of metastatic bladder cancer and/or less time to metastasis and/or lower survival rate and/or faster progression of bladder cancer and/or an increased risk of relapse of bladder cancer.

3. The method of claim 1 or 2, wherein the prognostic score is defined such that a higher activity is more associated with muscle invasive bladder cancer than with non-muscle invasive bladder.

4. A method of determining a response score indicating responsiveness of a subject diagnosed with bladder cancer to treatment with an inhibitor of the Notch cellular signaling pathway, the method comprising:
- receiving an activity of a Notch cellular signaling pathway determined in an urothelial cell sample obtained from the subject;
- comparing the received activity to one or more reference activities of the Notch cellular signaling pathway, and
- determining the response score,
- wherein the response score is defined such that the activity is positively correlated with responsiveness.

5. The method of claim 4, further comprising stratifying the subject between at least two groups of responders and non-responders.

6. The method of any of the preceding claims, further comprising providing the determined prognostic score and the determined response score, respectively, so as to make a prognosis or to predict responsiveness, respectively.

7. The method of any of the preceding claims, wherein the step of comparing the received activity to one or more reference activities of the Notch cellular signaling pathway comprises evaluating the received activity against a calibrated model relating the one or more reference activities to respective one or more reference prognostic scores and respective one or more reference response scores, respectively.

8. The method of any of the preceding claims, wherein the activity of the Notch cellular signaling pathway is defined to be related to an activity level of a Notch cellular signaling pathway associated transcription factor (TF) element.

9. The method of any of the preceding claims,
wherein the activity of the Notch cellular signaling pathway is inferred or inferable by a method comprising:
- receiving expression levels of three or more target genes of the Notch cellular signaling pathway,
- determining an activity level of a Notch cellular signaling pathway associated transcription factor (TF) element, the TF element controlling transcription of the three or more target genes, the determining being based on evaluating a calibrated mathematical pathway model relating the expression levels of the three or more target genes to the activity level of the TF element, and
- inferring the activity of the Notch cellular signaling pathway based on the determined activity level of the Notch cellular signaling pathway associated TF element.

10. The method of any of the preceding claims, wherein the three or more Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

11. An apparatus for determining a prognostic score indicating prognosis for a subject diagnosed with bladder cancer or determining a response score indicating responsiveness of a subject diagnosed with bladder cancer to treatment with an inhibitor of the Notch cellular signaling pathway, the apparatus comprising a digital processor configured to perform the method of any of claims 1 to 10.

12. A non transitory storage medium for determining a prognostic score indicating prognosis for a subject diagnosed with bladder cancer or determining a response score indicating responsiveness of a subject diagnosed with bladder cancer to treatment with an inhibitor of the Notch cellular signaling pathway, the non transitory storage medium storing instructions that are executable by a digital processing device to perform the method of any of claims 1 to 10.

13. A computer program for determining a prognostic score indicating prognosis for a subject diagnosed with bladder cancer or determining a response score indicating responsiveness of a subject diagnosed with bladder cancer to treatment with an inhibitor of the Notch cellular signaling pathway, the computer program comprising program code means for causing a digital processing device to perform a method of any of claims 1 to 10, when the computer program is run on the digital processing device.

14. A kit for determining a prognostic score indicating prognosis for a subject diagnosed with bladder cancer or determining a response score indicating responsiveness of a subject diagnosed with bladder cancer to treatment with an inhibitor of the Notch cellular signaling pathway, the kit comprising:
- components for determining the expression levels of at least three target genes of a Notch cellular signaling pathway, and
- the apparatus of claim 11, the non-transitory storage medium of claim 12, or the computer program of claim 13.

15. Use of a kit comprising components for determining the expression levels of at least three target genes of a Notch cellular signaling pathway for determining a prognostic score indicating prognosis for a subject diagnosed with bladder cancer or determining a response score indicating responsiveness of a subject diagnosed with bladder cancer to treatment with an inhibitor of the Notch cellular signaling pathway.
